# EUROPÄISCHE PATENTANMELDUNG

(11) **EP 1 020 183 A2**
(43) Veröffentlichungstag der Anmeldung: **19.07.2000**
(21) Anmeldenummer: 99125471.5
(22) Anmeldetag: 21.12.1999
(51) Int. Cl.: A61K 9/22, A61K 9/36, A61K 9/52, A61K 31/135, A61P 25/04

(54) **Analgetikum mit kontrollierter Wirkstofffreisetzung**

(30) Priorität: 18.01.1999 DE 19901683
(71) Anmelder: Grünenthal GmbH, 52078 Aachen (DE)
(72) Erfinder: Betzing, Jürgen, Dr., 79539 Lörrach-Tumringen (DE); Bartholomäus, Johannes, Dr., 52080 Aachen (DE)

(57) **Zusammenfassung**

Die Erfindung betrifft ein oral zu verabreichendes Präparat mit einer kontrollierten Freisetzung wenigstens eines Analgetikums aus Mikrotabletten mit einem Durchmesser von < 3 mm.

## Beschreibung

Die vorliegende Erfindung betrifft eine Arzneimittelformulierung, aus der der analgetische Wirkstoff kontrolliert freigesetzt wird.

Aus dem Stande der Technik sind eine Vielzahl von Formulierungen analgetischer Schmerzmittel bekannt, die eine kontrollierte Freisetzung des Wirkstoffes gewährleisten.

So wird u. a. in der EP-A-0647448 bereits ein analgetisch wirksames Präparat mit einer verzögerten Wirkstoffabgabe beschrieben, das aus einer Vielzahl, in retardierter Form vorliegender, opioidhaltiger Substrate mit einem Durchmesser von 0,1 bis 3 mm als eine Eintagesdosis besteht. Dafür geeignete Substrate können in Form von Sphäroiden, Mikrokugeln, Pellets oder Granulate vorliegen. Um diese Art von Substraten herzustellen, sind in aller Regel relativ aufwendige Formulierungstechniken, wie z. B. das Schichtaufbauverfahren für Pellets oder das Extrusions-, Sphäronisationsverfahren für Sphäroide, notwendig.

Darüber hinaus besteht Bedarf bei vielen therapeutischen Anwendungen ein Analgetikum enthaltendes Arzneimittels individuell zu dosieren, wie dies bei oral zu verabreichenden, flüssigen Darreichungsformen in Form von Tropfen möglich ist, und bei dessen Herstellung auf konventionelle unkomplizierte Formulierungsverfahren, wie die Tablettierung, zurückgreifen zu können.

Aufgabe der vorliegenden Erfindung war es daher, ein oral zu verabreichendes Präparat mit einer kontrollierten Freisetzung wenigstens eines Analgetikums zur Verfügung zu stellen, das eine individuelle, exakte, der Abgabe von Tropfen vergleichbare Dosierung aus z. B. Vorratsbehältern erlaubt oder die Aufteilung einer bestimmten Wirkstoffmenge auf eine leicht und genau zu kontrollierende Anzahl von Substraten ermöglicht, und das nach gängigen, wenig aufwendig Formulierungstechniken hergestellt werden kann.

Erfindungsgemäß wird diese Aufgabe gelöst durch Bereitstellung
eines oral zu verabreichenden Präparates mit einer kontrollierten Freisetzung wenigstens eines Analgetikums aus einer Mikrotablette mit einem Durchmesser < 3 mm.

Vorzugsweise weisen diese Mikrotabletten einen Durchmessern von 1 bis 3 mm, besonders bevorzugt von 1,5 bis 3 mm, auf.

Als analgetischen Wirkstoff enthalten die erfindungsgemäßen Mikrotabletten vorzugsweise wenigstens ein Opioid. Vorzugsweise wird als Opioid Hydromorphon, Oxycodon, Morpliin, Levorphanol, Methadon, Dihydrocodein, Codein, Fentanyl, Dihydromorphin, Pethidin, Piritramid, Buprenorphin, Tilidin, Tramadol, deren jeweilige Salze oder deren Mischungen eingesetzt.

Ganz besonders bevorzugt wird als Analgetikum Tramadol, Tramadol-Hydrochlorid, Morphin, Morphin-Hydrochlorid und/oder Morphin-Sulfat verwendet.

Neben den genannten opioiden Analgetika können nicht opioide Analgetika in dem erfindungsgemäßen Präparat vorhanden sein, die mit den opioiden Analgetika gegebenenfalls eine synergistische Wirkung zeigen. Zu diesen nicht opioiden Analgetika gehören Ibuprofen, Ketoprofen, Flurbiprofen, Paracetamol, Naproxen, Propyphenazon, Acemetacin, Actylsalicylsäure, Metamizol und/oder deren jeweilige Salze.

Die erfindungsgemäß zum Einsatz kommenden Mikrotabletten zeichnen sich durch eine kontrollierte Freisetzung des Analgetikums aus.

Unter kontrollierter Freisetzung des Analgetikums wird sowohl eine nicht verzögerte als auch eine retardierte Freisetzung verstanden. Vorzugsweise wird der opioide Wirkstoff retardiert freigesetzt.

Diese Freisetzung kann erreicht werden, indem der Wirkstoff in eine Retardmatrix eingebunden wird. Durch die Einbettung in ein Matrixmaterial wird erreicht, daß eine kontrolliert, verzögerte Freisetzung des Wirkstoffes über den angestrebten Zeitraum erreicht wird. Vorzugsweise wird dabei angestrebt, die Freigabe des Wirkstoffes so einzustellen, daß pro 24 Stunden eine zweimalige, vorzugsweise nur eine einmalige Einnahme ausreicht.

Als Matrixmaterialien sind pharmazeutisch verträgliche, hydrophile Materialien geeignet, die dem Fachmann bekannt sind. Vorzugsweise werden als hydrophile Matrixmaterialien Polymere, wie z. B. Celluloseether, Celluloseester oder Acrylharze verwendet. Ganz besonders bevorzugt sind als Matrixmaterialien Ethylcellulose, Hydroxypropylmethylcellulose, Hydroxypropylcellulose, Hydroxymethylcellulose, Poly(meth)acrylsäure und/oder, deren Derivate, wie deren Salze, Amide oder Ester.

Das Matrixmaterial kann aber auch aus hydrophoben Materialien, wie z. B. hydrophoben Polymeren, Wachsen, Fetten, Ölen, langkettigen Fettsäuren, Fettalkoholen oder entsprechenden Estern oder deren Gemischen bestehen. Vorzugsweise werden als hydrophobe Materialien Mono- oder Diglyceride von C12-C30-Fettsäuren und/oder C12-C30-Fettalkohole und/oder Wachse eingesetzt.

Es ist auch möglich, eine Mischung der genannten hydrophilen und hydrophoben Materialien als retardierendes Matrixmaterial einzusetzen.

Darüber hinaus können die erfindungsgemäßen Mikrotabletten als weitere Bestandteile pharmazeutisch übliche Hilfsstoffe wie Füllstoffe, beispielsweise Laktose, mikrokristalline Cellulose oder Calciumhydrogenphosphat, sowie Gleit-, Schmier- und Fließregulierungsmittel, wie beispielsweise hochdisperses Siliciumdioxid, Talkum, Magnesiumsterat und/oder Stearinsäure, enthalten.

Besonders bevorzugt wird als pharmazeutisch verträgliches Matrixmaterial mindestens ein Celluloseether und/oder Celluloseester, dessen 2 gew.%ige wässrigen Lösung bei 20°C eine Viskosität von 3000 bis 150000 mPas, vorzugsweise von 10000 bis 150000 mPas aufweist, gegebenenfalls in Kombination mit einem im wässrigen Medium nicht quellbaren Füllstoff, wie beispielsweise Calciumhydrogenphosphat, oder mit einem unlöslichen, im wässrigen Medium quellbaren Füllstoff, wie beispielsweise mikrokristalline cellulose, oder einem in wässrigen Medien löslichen Füllstoff wie beispielsweise Lactose.

Der Gehalt an Analgetikum, vorzugsweise an opioidem Analgetikum, wird in Abhängigkeit von der gewünschten Freisetzungsdauer und freizusetzenden Menge des Analgetikums eingestellt. Vorzugsweise liegt der Wirkstoffgehalt zwischen 10 und 85 Gew.%, besonders bevorzugt zwischen 25 und 70 Gew-%, bezogen auf die Gesamtmischung. Dem Fachmann ist aufgrund der Wirkung der opioiden bzw. nicht opioiden Analgetika bekannt, in welchen Mischungsverhältnissen diese einzusetzen sind, damit die gewünschte Freisetzung an Wirkstoffen erreicht wird.

Bei den erfindungsgemäßen Präparaten, die sich aus Mikrotabletten zusammensetzen, kann eine kontrollierte Abgabe des Wirkstoffes auch erreicht werden, indem die einzelnen Tabletten mit wenigstens einem Überzug, der eine kontrollierte, in der Regel retardierte Freigabe des Wirkstoffes in einem wässrigen Medium gestattet, überzogen werden. Geeignete Retardüberzüge umfassen wasserunlösliche Wachse oder Polymere, wie z. B, Acrylharze, vorzugsweise Poly(meth)acrylate oder wasserunlösliche Cellulosen, vorzugsweise Ethylcellulose. Diese Materialien sind aus dem Stande der Technik, z. B. Bauer, Lehmann, Osterwald, Rothgang "Überzogene Arzneiformen", Wissenschaftliche Verlagsgesellschaft mbH Stuttgart, 1998, Seite 69 ff., bekannt und werden hiermit als Referenz eingeführt.

Neben den wasserunlöslichen Polymeren können gegebenenfalls zur Einstellung der Freisetzungsrate des Wirkstoffes auch nicht retardierende, vorzugsweise wasserlösliche Polymere in Mengen bis 30 Gew.%, wie beispielsweise Polyvinylpyrrolidon oder wasserlösliche Cellulosen, vorzugsweise Hydroxypropylmethylcellulose oder Hydroxypropylcellulose, und/oder die bekannten Weichmacher mitverwendet werden.

Neben dem retardierenden Überzug können die erfindungsgemäßen Mikrotabletten noch mit weiteren Überzügen ausgerüstet werden. So ist es möglich, einen den Wirkstoff enthaltenden Überzug aufzubringen, aus dem der Wirkstoff unretardiert nach der oralen Verabreichnung des Präparates freigesetzt wird. Solche Mehrschichtenmikrotabletten können nach der Verabreichung sehr rasch eine Initialdosis des Analgetikums zur Schmerzlinderung zur Verfügung stellen, wobei das Niveau des Analgetikums durch die anschließende retardierte Abgabe des Wirkstoffes gehalten werden kann.

Desweiteren können die Mikrotabletten neben dem Retard-Überzug auch einen Überzug aufweisen, der sich pH-abhängig auflöst. So kann erreicht werden, daß z. B. eine bestimmte Anzahl der Mikrotabletten eines Präparates den Magentrakt unaufgelöst passiert und erst im Darmtrakt Wirkung zeigt.

Eine weitere bevorzugte Ausführungsform der erfindungsgemäßen Präparate besteht darin, daß die mit einem retardierten und gegebenenfalls weiteren Überzügen ausgerüsteten Mikrotabletten bereits den Wirkstoff in einer Matrix enthalten, die eine kontrollierte, verzögerte Freisetzung des Wirkstoffes garantiert, oder die matrixretardierten Mikrotabletten keinen Retard-Überzug, aber mindestens einen der genannten Überzüge aufweisen, die eine Initialdosis und/oder eine pH-abhängige Freisetzung gewähren.

Die Herstellung der Mikrotabletten erfolgt nach bekannten Methoden, wie sie z. B. in EP-A-0166315 beschrieben sind. Die entsprechende Offenbarung wird hiermit als Referenz eingeführt.

Vorzugsweise erfolgt die Herstellung der Mikrotabletten, indem alle Bestandteile der Tablette, vorzugsweise durch ein 0,6 mm Sieb gesiebt und dann homogen vermischt werden. Die Mischung kann in ein Granulat überführt werden, wobei der Siebschritt dann vorzugsweise nach der Granulation durchgeführt wird. Im Fall der Granulation werden vorzugsweise vor dem Verpressen Gleit- und/oder Schmiermittel zugemischt. Die homogene Mischung wird auf einer Tablettenpresse, vorzugsweise einer Rundläufertablettenpresse, zu Tabletten mit einem Durchmesser von 1 bis 3 mm, vorzugsweise 1,5 bis 3 mm, verpreßt. Diese Methode wird vorzugsweise auch bei der Herstellung von Mikrotabletten mit einer Matrixretardierung durchgeführt, wobei für hydrophobe, bei < 100°C schmelzbare Matrixmaterialien die Schmelzgranulation ein bevorzugtes Herstellungsverfahren darstellt. Geeignete Methoden hierfür sind dem Fachmann bekannt.

Sofern die erfindungsgemäßen Präparate Mikrotabletten mit Überzügen enthalten, können diese nach ublichen Verfahren, wie durch z. B. Dragieren, Aufsprühen von Lösungen, Dispersionen oder Suspensionen, durch Schmelzverfahren oder durch Pulverauftragverfahren aufgebracht werden.

Die oral zu verabreichenden, erfindungsgemäßen Präparate bestehend aus Mikrotabletten weisen außerdem den großen Vorteil auf, daß die gewünschte Dosis an Analgetikum auf eine mit wenig Aufwand abzählbare Anzahl von Einheiten verteilbar ist. Dadurch ist es möglich, das oral zu verabreichende Präparat entsprechend den individuellen Bedürfnissen eines Patienten zusammenzustellen, indem man z. B. die gewünschte Anzahl von Mikrotabletten mit Hilfe einer Dosiereinheit, vorzugsweise einem Spender, entsprechend der individuell zu erreichenden Freisetzungsdauer und freizusetzenden Menge an Analgetikum aus einem Mikrotablettenvorrat entnimmt.

Ein weiterer Gegenstand der Erfindung sind daher individuell dosierbare, oral zu verabreichende Präparate, deren Anzahl an Mikrotabletten entsprechend der individuell gewünschten Freisetzungsdauer und freizusetzenden Menge an Analgetikum bestimmt wird.

Ein weiterer Gegenstand der Erfindung ist auch die Bereitstellung der erfindungsgemäßen, oral zu verabreichenden Präparate in Kapseln, in denen die Mikrotabletten mit einer kontrollierten Freisetzung des Analgetikums in einer definierten Anzahl entsprechend der individuell zu erreichenden Freisetzungsdauer und freizusetzenden Menge an Analgetikum vorhanden sind. Vorzugsweise wird die Anzahl von Mikrotabletten in einer Kapsel so gewählt, daß die Dosis für eine einmalige oder zweimalige Applikation pro Tag ausreicht. Auch bei dieser Darreichungsform ist es vorteilhaft, daß die Dosis des Analgetikums auf eine mit wenig Aufwand abzählbare Anzahl von Mikrotabletten verteilt ist, dem Patienten aber durch die in einer Kapsel festgelegte Dosis das Zählen abgenommen wird.

Darüber hinaus können die erfindungsgemäßen, oral zu verabreichenden Präparate auch in einer sogenannten Makrotablette, d. h. einer Tablette mit üblichem Ausmaß, vorliegen, zu der eine bestimmte Anzahl von Mikrotabletten entsprechend der individuell zu erreichenden Freisetzungsdauer und freizusetzenden Menge an Analgetikum mit den üblichen Hilfs- und Zusatzstoffen für Tabletten zu einer Tablette verpreßt sind. Auch hier ist es vorteilhaft, wenn die Anzahl der Mikrotabletten, aus der sich die Makrotablette zusammensetzt, so gewählt wird, daß die zu erreichende Freisetzungsdauer und freizusetzende Menge des Analgetikums für eine einmalige oder zweimalige Applikation pro Tag ausreicht.

Ein weiterer Gegenstand der vorliegenden Erfindung sind daher auch oral zu verabreichende Präparate mit einer kontrollierten Freisetzung wenigstens eines Analgetikums aus Mikrotabletten, wobei eine bestimmte Anzahl von Mikrotabletten entsprechend der individuell zu erreichenden Freisetzungsdauer und freizusetzenden Menge des Analgetikums mit üblichen Hilfs- und Zusatzstoffen zu einer Tablette verpreßt sind.

### Beispiele

Das Freisetzungsprofil der in den Beispielen hergestellten Präparate wurde wie folgt bestimmt:

Die Zubereitungen wurden in einen Drehkörbchenapparat (gemäß Europäischem Arzneibuch) bei einer Temperatur des Freisetzungsmediums von 37°C, einer Umdrehungsgeschwindigkeit des Drehkörbchens von 100 Minuten-1 in 600 ml künstlichem Magensaft (pH 1,2) gegeben. Nach 120 min wurde der pH-Wert des Freisetzungsmediums durch Zugabe von Phosphat-Pufferlösung auf pH 7,2 erhöht. Dieser pH-Wert wurde bis zu den Ende der Untersuchung beibehalten. Die jeweils zu einem Zeitpunkt freigesetzte Menge des Wirkstoffes wurde spektralphotonisch bestimmt .

### Beispiel 1:

### Zusammensetzung:

| | | pro Tablette | pro Kapsel mit. 10 Tabletten |
|---|---|---|---|
| Komponenten der Mikrotabletten | Tramadol HCl | 10,0 mg | 100 mg |
| | Mikrokristalline Cellulose | 4,0 mg | 40 mg |
| | Povidon ® K30 | 0,8 mg | 8 mg |
| | Magnesiumstearat | 0,2 mg | 2 mg |
| | | | |
| Total | | 15 mg | 150 mg |

Tramadolsalz und mikrokristalline Cellulose wurden mit einer wässrigen Lösung von Povidon ® K30 granuliert, getrocknet, gesiebt, mit Magnesiumstearat vermischt, zu Tabletten mit 3 mm Durchmesser und ca. 2 mm Höhe verpreßt und in Kapseln mit je 10 Tabletten abgefüllt.

Das Freisetzungsprofil, war wie folgt:
nach 15 Minuten > 80 % Wirkstofffreisetzung

### Beispiel 2:

### Zusammensetzung:

| | | pro Tablette | pro Kapsel mit 10 Tabletten |
|---|---|---|---|
| Komponenten der Mikrotabletten | Tramadol HCl | 10,0 mg | 100 mg |
| | Mikrokristalline Cellulose | 4,0 mg | 40 mg |
| | Povidon K 30 | 0,8 mg | 8 mg |
| | Magnesiumstearat | 0,2 mg | 2 mg |
| Überzugskomponenten | Ethylcellulose (Aquacoat ®) | 0,8 mg | 8 mg |
| | Dibutylsebacat | 0,2 mg | 2 mg |
| | | | |
| Total | | 16,0 mg | 160 mg |

Tramadolsalz und mikrokristalline Cellulose wurden mit einer wässrigen Lösung von Povidon K 30 (Polyvinylpyrrolidon) granuliert, getrocknet, gesiebt, mit Magnesiumstearat vermischt, zu Tabletten mit 3 mm Durchmesser verpreßt und mit wässriger Ethylcellulose/Dibutylsebacat **Dispersion im Mengenverhältnis 4:1 in einer Wirbelschichtapparatur durch Aufsprühen der Dispersion unter kontinuierlichem Trocknen überzogen. Es wurden jeweils 10 Mikrotabletten in eine Kapsel abgefüllt.**

Das durchschnittliche Freisetzungsprofil war:

| Zeit nach | Wirkstofffreisetzung % der ursprünglichen Wirkstoffkonzentration |
|---|---|
| 30 Minuten | 1 % |
| 240 Minuten | 18 % |
| 480 Minuten | 29 % |

### Beispiel 3:

Es wurden Mikrotabletten mit 2 mm Durchmesser und ca 2 mm Höhe mit folgender Zusammensetzung analog Beispiel 2 hergestellt und überzogen. Jeweils 20 Tabletten wurden in eine Kapsel abgefüllt.

| | | pro Tablette | pro Kapsel mit 20 Tabletten |
|---|---|---|---|
| Komponenten der Mikrotabletten | Tramadol HCl | 5,0 mg | 100 mg |
| | Mikrokristalline Cellulose | 2,0 mg | 40 mg |
| | Povidon K 30 | 0,4 mg | 8 mg |
| | Magnesiumstearat | 0,1 mg | 2 mg |
| Überzugskomponenten | Ethylcellulose (Aquacoat®) | 0,4 mg | 8 mg |
| | Dibutylsebacat | 0,1 mg | 2 mg |
| | | | |
| Total | | 8,0 mg | 160 mg |

### Beispiel 4:

Zur Herstellung von Mikrotabletten mit einer Matrixretardierung wurden Tramadol HCl (5 mg/Tablette) und Glycerylbehenat (Compritol 880 ato ®) (5 mg/Tablette) durch ein Sieb mit 0,6 mm Maschengröβe abgesiebt. Die homogenisierte Mischung wurde anschließend mit Hilfe von 2 mm Stempeln zu entsprechenden Mikrotabletten verpreßt. Diese zeigten folgendes Freisetzungsprofil:

| Zeit nach | Wirkstofffreisetzung in % der ursprünglichen Wirkstoffkonzentration |
|---|---|
| 60 Minuten | 40 % |
| 120 Minuten | 60 % |
| 240 Minuten | 75 % |
| 480 Minuten | 85 % |

## Patentansprüche

1. Oral zu verabreichendes Präparat mit einer kontrollierten Freisetzung wenigstens eines Analgetikums aus Mikrotabletten mit einem Durchmesser von < 3 mm.

2. Präparat nach Anspruch 1, dadurch gekennzeichnet, daß die Mikrotablette einen Durchmesser von 1 bis 3 mm, vorzugsweise 1,5 bis 3 mm, haben.

3. Präparat nach Anspruch 1 oder 2, dadurch gekennzeichnet, daß das Analgetikum wenigstens ein Opioid ist.

4. Präparat nach Anspruch 3, dadurch gekennzeichnet, daß als Opioid Hydromorphon, Oxycodon, Morphin, Levorphanol, Methadon, Dihydrocodein, Fentanyl, Codein, Dihydromorphin, Pethidin, Piritramid, Buprenorphin, Tilidin, tramadol, deren jeweilige Salze oder deren Mischungen eingesetzt wird.

5. Präparat nach Anspruch 4, dadurch gekennzeichnet, daß als Opioid Tramadol, Tramadol-Hydrochlorid, Morphin, Morphin-Hydrochlorid und/oder Morphin-Sulfat eingesetzt wird.

6. Präparat nach einem oder mehreren der Ansprüche 1 bis 5, dadurch gekennzeichnet, daß die Mikrotabletten das Analgetikum in einer retardierenden Matrix gleichmäßig verteilt aufweisen.

7. Präparat nach Anspruch 6, dadurch gekennzeichnet, daß die Matrix wenigstens ein Polymeres, ein Wachs, ein Fett, ein Öl, eine Fettsäure, einen Fettalkohol oder einen entsprechenden Ester aufweist.

8. Präparat nach Anspruch 7, dadurch gekennzeichnet, daß als Polymere Celluloseether, Celluloseester und/oder Acrylharze eingesetzt werden.

9. Präparat nach einem der Ansprüche 6 bis 8, dadurch gekennzeichnet, daß als Matrixmaterial Ethylcellulose, Hydroxypropylmethylcellulose, Hydroxypropylcellulose, Hydroxymethylcellulose, Mono- und/oder Dyglyderide von C₁₂-C₃₀ Fettsäuren und/oder C₁₂-C₃₀ Fettalkoholen eingesetzt werden

10. Präparat nach einem der Ansprüche 1 bis 5, dadurch gekennzeichnet, daß die Mikrotabletten mit wenigstens einem Überzug versehen sind.

11. Präparat nach Anspruch 10, dadurch gekennzeichnet daß der Überzug retardierend ist.

12. Präparat nach Anspruch 10 oder 11, dadurch gekennzeichnet, daß der Überzug auf einem wasserunlöslichen Polymeren oder Wachs basiert.

13. Präparat nach Anspruch 12, dadurch gekennzeichnet, daß als Polymeres ein Acrylharz oder Cellulosederivat, vorzugsweise Alkylcellulose, eingesetzt wird.

14. Präparat nach Anspruch 13, dadurch gekennzeichnet, daß Ethylcellulose und/oder ein Poly(meth)acrylat als Überzugsmaterial eingesetzt wird.

15. Präparat nach einem oder mehreren Ansprüchen 1 bis 14, dadurch gekennzeichnet, daß die Mikrotabletten in einer Kapsel vorliegen.

16. Präparat nach Anspruch 15, dadurch gekennzeichnet, daß in der Kapsel jeweils eine definierte Anzahl von Mikrotabletten entsprechend der individuell zu erreichenden Freisetzungsdauer und freizusetzenden Menge an Analgetikum enthalten ist.

17. Präparat nach Anspruch 16, dadurch gekennzeichnet, daß die Anzahl der Mikrotabletten in der Kapsel für eine 1x oder 2x Applikation pro Tag reicht.

18. Präparat nach einem Ansprüche 1 bis 14, dadurch gekennzeichnet , daß die Mikrotablette(n) mit Hilfe einer Dosiereinheit, vorzugsweise einem Spender, in einer abzählbaren Anzahl entsprechend der individuell zu erreichenden Freisetzungsdauer und freizusetzenden Menge an Analgetikum aus einem Mikrotablettenvorrat entnehmbar sind.

19. Präparat nach einem oder mehreren Ansprüchen 1 bis 14, dadurch gekennzeichnet, daß eine bestimmte Anzahl von Mikrotabletten entsprechend der individuell zu erreichenden Freisetzungsdauer und freizusetzenden Menge an Analgetikum mit üblichen Hilfs- und Zusatzstoffen zu einer Tablette verpreßt sind.

20. Präparat nach einem oder mehreren Ansprüchen 1 bis 5, dadurch gekennzeichnet, daß das Analgetikum innerhalb von 30 Minuten zu mehr als 75 % freigesetzt wird.
